# EUROPÄISCHE PATENTANMELDUNG

(11) **EP 3 205 357 A1**
(43) Veröffentlichungstag der Anmeldung: **16.08.2017**
(21) Anmeldenummer: 16197303.7
(22) Anmeldetag: 04.11.2016
(51) Int. Cl.: A61L 2/18, A61L 2/24, A47L 15/42

(54) **REINIGUNGS- UND DESINFEKTIONSGERÄT**

(71) Anmelder: MELAG Medizintechnik oHG, 10829 Berlin (DE)
(72) Erfinder: FRIEDRICH, Frank, 16341 Panketal (DE); LITZBA, Guido, 14513 Teltow (DE); HÖRNING, Andreas, 10969 Berlin (DE)
(74) Vertreter: Maikowski & Ninnemann Patentanwälte Partnerschaft mbB

(57) **Zusammenfassung**

Die Erfindung betrifft ein Reinigungs- und Desinfektionsgerät für medizinische und/oder zahnmedizinische Instrumente, mit einer an einer Gerätefront angeordneten Gerätetür, einer Wasserenthärtungseinrichtung und einem Salzbehälter (3, 30), in welchem eine Salzsole enthalten sein kann, die zu einer Regeneration eines in der Wasserenthärtungseirichtung befindlichen Enthärtungsmittels dient. Erfindungsgemäß ist vorgesehen, dass der Salzbehälter (3, 30) eine von einer Außenseite der Gerätefront des Reinigungs- und Desinfektionsgeräts (1, 10) zugängliche Salzeinfüllöffnung (12) aufweist, durch die hindurch Salz in den Salzbehälter (3, 30) eingefüllt werden kann, ohne dass die Gerätetür hierfür geöffnet werden muss, wobei ein Einfüllen von Salz in die Salzeinfüllöffnung (12) während eines Programmlaufs des Reinigungs- und Desinfektionsgeräts (1, 10) oder in einem ausgeschalteten Zustand des Reinigungs- und Desinfektionsgeräts (1, 10) möglich ist. Die Erfindung betrifft ferner ein Verfahren zum Betrieb eines solchen Reinigungs- und Desinfektionsgeräts.

## Beschreibung

Die Erfindung betrifft ein Reinigungs- und Desinfektionsgerät für medizinische und/oder zahnmedizinische Instrumente gemäß dem Oberbegriff des Anspruchs 1.

Reinigungs- und Desinfektionsgeräte enthalten häufig eine integrierte Enthärtungsanlage zur Enthärtung von Wasser durch Ionenaustausch. Innerhalb der geräteinternen Enthärtungsanlage kommt ein Harz zum Einsatz, das sich in Abhängigkeit der Menge des erzeugten enthärteten Wassers erschöpft. Zur Regeneration des Harzes und zur Aufrechterhaltung der Enthärtungsfunktion muss die Enthärtungsanlage regelmäßig mit einer Salzsole durchströmt werden, weshalb sich innerhalb des Geräts ein mit Salz befüllter Salzbehälter befindet.

Da sich bei der Erzeugung der Salzsole das Salz relativ schnell verbraucht, muss es durch den Anwender regelmäßig nachgefüllt werden. Problematisch dabei ist die Tatsache, dass sich in dem Salzbehälter stets Salzsole befindet, die durch das Einfüllen von Salz verdrängt wird. Somit kommt es zu einem Überlaufen der Salzsole, welche extrem korrosive Eigenschaften besitzt.

Sowohl bei handelsüblichen Geschirrspülern als auch bei Reinigungs- und Desinfektionsgeräten wird das Salz in den meisten Fällen über die Waschkammer des Geräts nachgefüllt. Dazu muss der Anwender meist einen Schraubverschluss im Waschkammerboden öffnen und durch diesen das Salz aus einem Beutel in den Salzbehälter einfüllen. Somit bleibt die überlaufende Salzsole innerhalb des Geräts und kann nach dem Befüllen manuell oder automatisch beseitigt werden. Dies sollte jedoch möglichst kurzfristig nach dem Überlaufen der Salzsole erfolgen, damit keine Korrosion durch die Salzsole in der Waschkammer auftritt.

Aus der EP 1 457 153 B1 ist eine Geschirrspülmaschine mit einem Wasserenthärter bekannt, dessen Salzbehälter in der Gerätetür angeordnet ist. Dabei wird der Salzbehälter durch eine Einfüllöffnung, die auf der Innenseite der Gerätetür angeordnet ist, befüllt. Es ist also erforderlich, die Gerätetür zu öffnen, um den Salzbehälter mit Salz zu befüllen. Wenn sich die Gerätetür während des Befüllvorgangs versehentlich schließt oder ihre Lage anderweitig ändert, kann es leicht zu einem Verschütten von Salz kommen.

Die DE 103 12 744 A1 beschreibt eine Geschirrspülmaschine, die eine Einrichtung zur Luftzufuhr in den Salzbehälter der Geschirrspülmaschine aufweist. Dadurch soll ein Überlaufen von Salzsole beim Befüllen des Salzbehälters vermieden werden. Diese deutsche Patentanmeldung beschreibt zudem die grundsätzliche Möglichkeit, den Salzbehälter so anzuordnen, dass er von außen befüllbar ist, ohne hierfür jedoch konkrete Lösungsvorschläge zu unterbreiten.

Aus der EP 2 798 110 B1 ist eine Waschmaschine bekannt, bei der ein Salzbehälter hinter einer Dosiereinrichtung für Waschmittel und Weichspüler angeordnet ist, wobei der Salzbehälter zusammen mit der Dosiereinrichtung bewegt und aus der Waschmaschine entnommen werden kann. Dies kann allerdings nicht während eines laufenden Programms der Waschmaschine erfolgen, da bei entnommener Dosiereinrichtung weder das Waschmittel noch der Weichspüler in die Waschtrommel eingebracht werden können noch das zum Waschen verwendete Wasser korrekt in die Waschtrommel einfließen kann.

Aus der DE 10 2004 057 242 A1 ist eine Geschirrspülmaschine bekannt, die eine Einfüllöffnung für einen Salzbehälter auf dem Boden einer Waschkammer aufweist. Diese Einfüllöffnung ist mit Seitenwänden versehen, um das Einfüllen von Salz zu erleichtern. Ferner ist die Einfüllöffnung in einem solchen Abstand zu der Tür der Geschirrspülmaschine angeordnet, dass sie bei geschlossener Tür sicher verriegelt ist und nur bei geöffneter Tür geöffnet werden kann.

Aus der DE 10 2004 057 243 A1 ist eine ähnliche Anordnung bekannt. So wird in dieser deutschen Patentanmeldung eine Geschirrspülmaschine beschrieben, bei der der Deckel eines Salzbehälters derart geformt ist, dass er bei geschlossener Tür der Geschirrspülmaschine nicht versehentlich geöffnet werden kann. Folglich muss zum Einfüllen von Salz in den Salzbehälter erst die Tür der Geschirrspülmaschine geöffnet werden, um dann in ergonomisch ungünstiger Art und Weise Salz in den Geschirrspüler einfüllen zu können.

Der vorliegenden Erfindung liegt die Aufgabe zugrunde, ein Reinigungs- und Desinfektionsgerät bereitzustellen, bei dem ein vereinfachtes Einfüllen von Salz unabhängig vom konkreten Betriebszustand des Geräts möglich ist und bei dem die Korrosionsgefahr innerhalb der Waschkammer des Geräts beim Einfüllen von Salz reduziert ist. Ferner soll ein geeignetes Betriebsverfahren für ein solches Gerät bereitgestellt werden.

Diese Aufgabe wird mit einem Reinigungs- und Desinfektionsgerät mit den Merkmalen des Anspruchs 1 gelöst. Ein solches Gerät ist zur Reinigung und Desinfektion medizinischer und/oder zahnmedizinischer Instrumente vorgesehen. Es weist eine Gerätetür auf, die an einer Gerätefront angeordnet ist. Durch Öffnen der Gerätetür wird eine Waschkammer zugänglich, in die die medizinischen und/oder zahnmedizinischen Instrumente eingelegt werden können. Ferner weist das Gerät eine Wasserenthärtungseinrichtung und einen Salzbehälter auf. In dem Salzbehälter kann eine Salzsole enthalten sein. Beispielsweise ist es möglich, dass die Salzsole in dem Salzbehälter während des Betriebs des Geräts hergestellt wird. Die Salzsole wird während des Betriebs des Gerätes zur Regeneration eines Enthärtungsmittels eingesetzt, welches in der Wasserenthärtungseinrichtung vorgehalten wird.

Erfindungsgemäß zeichnet sich das Reinigungs- und Desinfektionsgerät dadurch aus, dass der Salzbehälter eine von einer Außenseite der Gerätefront des Reinigungs- und Desinfektionsgeräts zugängliche Salzeinfüllöffnung aufweist. Durch diese Salzeinfüllöffnung kann Salz in den Salzbehälter eingefüllt werden, ohne dass die Gerätetür hierfür geöffnet werden muss. Darüber hinaus ist ein Einfüllen von Salz in die Salzeinfüllöffnung unabhängig vom konkreten Betriebszustand des Reinigungs- und Desinfektionsgeräts möglich. Das heißt, das Salz kann gleichermaßen während eines Programmlaufs des Reinigungs- und Desinfektionsgeräts oder in einem ausgeschalteten Zustand des Reinigungs- und Desinfektionsgeräts eingefüllt werden. Dabei umfasst der Begriff "ausgeschalteter Zustand" auch einen Standby-Zustand (Ruhezustand), in dem das Reinigungs- und Desinfektionsgerät zwar nicht vollständig ausgeschaltet ist, jedoch kein Reinigungs- und/oder Desinfektionsprogramm durchführt.

Dass ein Einfüllen von Salz in die Salzeinfüllöffnung unabhängig vom konkreten Betriebszustand des Reinigungs- und Desinfektionsgeräts möglich ist, wird insbesondere dadurch erreicht, dass zum Einfüllen des Salzes keine für den Betrieb des Geräts essentiellen Bauteile des Geräts entnommen werden müssen. Vielmehr wird darauf geachtet, dass überhaupt keine Bauteile von dem Gerät entnommen werden oder aber dass entnommene Bauteile nicht für die Durchführung eines bestimmten Reinigungs- und Desinfektionsprogramms erforderlich sind. Dies wird im Detail weiter unten noch erläutert werden.

Der Salzbehälter kann beispielsweise in dem Reinigungs- und Desinfektionsgerät direkt hinter der Gerätefront in Nähe des Bodens oder unmittelbar auf dem Boden des Reinigungs- und Desinfektionsgeräts angeordnet sein. Dann ist es besonders einfach, den Salzbehälter von der äußeren Vorderseite des Gerätes her zu befüllen, ohne hierfür im Geräteinneren wie etwa in der Waschkammer oder an der Innenseite der Gerätetür bestimmte Tätigkeiten auszuführen, wie dies aus dem Stand der Technik bekannt ist.

Es ist auch nicht erforderlich, eine für das Einbringen eines Waschmittels bzw. für das korrekte Einlaufen von Wasser erforderliche Dosiereinrichtung aus dem Gerät zu entnehmen. Vielmehr kann der Zeitpunkt des Salzeinfüllens vom Benutzer frei gewählt werden, da keine geräteseitige Beschränkung hinsichtlich eines bestimmten Einfüllzeitpunkts mehr vorliegt. Im Ergebnis ist es dadurch möglich, Salz durch die Salzeinfüllöffnung in den Salzbehälter einzufüllen, wenn ein Reinigungs- und Desinfektionsprogramm bereits läuft, man jedoch erst beim Laufen des Programms feststellt, dass Salz nachgefüllt werden muss.

Bei dem in die Salzeinfüllöffnung einzufüllenden Salz handelt es sich typischerweise um klassisches Regeneriersalz, also reines Natriumchlorid in einer bestimmten, groben Körnung.

Um sowohl für ein ansprechendes Äußeres des Reinigungs- und Desinfektionsgeräts zu sorgen als auch eine leichte Zugänglichkeit der Salzeinfüllöffnung zu gewährleisten, ist die Salzeinfüllöffnung in einer Variante hinter einer an der Gerätefront ausgebildeten Klappe angeordnet. Diese Klappe kann dann, wenn Salz in die Salzeinfüllöffnung eingefüllt werden soll, einfach geöffnet werden.

Um für ein besonders einfaches Öffnen der Klappe in der Gerätefront zu sorgen, ist die Klappe in einer Variante derart ausgestaltet, dass sie werkzeugfrei geöffnet werden kann. Dann kann beispielsweise durch ein einfaches Ziehen an der Klappe bzw. durch das Ausüben eines Drucks auf die Klappe ein Öffnen der Klappe erreicht werden, sodass die dahinter angeordnete Salzeinfüllöffnung freigelegt wird.

In einer Variante weist der Salzbehälter eine Außenhülle auf, die zumindest in einem Abschnitt flexibel ausgebildet ist. Eine derartige Flexibilität kann zur bedarfsweisen Ausdehnung der Außenhülle verwendet werden, sodass das Innenvolumen des Salzbehälters vergrößert bzw. verkleinert werden kann. Dies ist insbesondere dann vorteilhaft, wenn in den Salzbehälter, der üblicherweise vollständig mit Salzsole gefüllt ist, weiteres Salz eingefüllt werden soll. Wenn dann aufgrund der zumindest teilweise flexiblen Außenhülle für eine Vergrößerung des für das Salz und die Salzsole zur Verfügung stehenden Volumens gesorgt wird, kann zusätzliches Salz in den Salzbehälter eingefüllt werden, ohne dass es zu einem Überlaufen von Salzsole kommt.

In einer weiteren Variante weist der Salzbehälter einen ersten Teil auf, der fest in dem Reinigungs- und Desinfektionsgerät angeordnet ist. Dieser erste Teil kann beispielsweise eine unflexible Außenhülle aufweisen. Ferner weist der Salzbehälter in dieser Variante einen zweiten Teil auf, der gegenüber dem ersten Teil beweglich ist. In dem zweiten Teil ist die Salzeinfüllöffnung angeordnet. Der zweite Teil kann beispielsweise auch eine starre, unflexible Außenhülle aufweisen. Alleine durch die grundsätzliche Beweglichkeit des zweiten Teils gegenüber dem ersten Teil kann erreicht werden, dass der zweite Teil mit der Salzeinfüllöffnung näher zu einem Benutzer hingebracht werden kann, sodass ein Einfüllen von Salz in die Salzeinfüllöffnung erleichtert wird. Grundsätzlich ist es auch denkbar, dass der erste Teil und/oder der zweite Teil des Salzbehälters aus einem flexiblen Außenmaterial bestehen, gleichwohl aber weiterhin gegeneinander beweglich angeordnet sind.

In einer weiteren Variante wird die Beweglichkeit zwischen dem ersten Teil des Salzbehälters und dem zweiten Teil des Salzbehälters dadurch erreicht, dass zwischen dem ersten Teil und dem zweiten Teil ein längenvariabler dritter Teil des Salzbehälters angeordnet ist. Dabei sind der erste Teil des Salzbehälters und der zweite Teil des Salzbehälters jeweils insbesondere fest mit dem dritten Teil des Salzbehälters verbunden. Eine Beweglichkeit des zweiten Teils gegenüber dem ersten Teil wird dann über die Längenveränderlichkeit des dritten Teils erreicht.

Eine besonders einfache Ausgestaltung des dritten Teils des Salzbehälters kann durch eine Realisierung in Form eines Faltenbalgs erreicht werden. Ein solcher Faltenbalg gewährleistet eine Veränderung der Länge des dritten Teils des Salzbehälters, wodurch der zweite Teil des Salzbehälters zu einem Benutzer hingezogen werden kann. Gleichzeitig vergrößert ein Faltenbalg in seinem ausgezogenen Zustand das Innenvolumen des Salzbehälters, sodass die oben erläuterten vorteilhaften Auswirkungen eines vergrößerten Innenvolumens in Bezug auf das Einfüllen von Salz ausgenutzt werden können. Gleichzeitig gestattet ein Faltenbalg auch ein einfaches Zusammenschieben des Salzbehälters, wenn nämlich der zweite Teil in Richtung auf den ersten Teil des Salzbehälters bewegt wird und der Faltenbalg wieder in seinen eingefahrenen Zustand überführt wird. Eine solche Bewegung wird insbesondere dann durchgeführt, wenn das Salz in die Salzeinfüllöffnung eingefüllt wurde und die Salzeinfüllöffnung anschließend verschlossen wurde. Denn dann führt ein Zusammenschieben des Salzbehälters zu einem Ausströmen von in dem Salzbehälter enthaltener Salzsole durch die dafür vorgesehenen Anschlussleitungen, nicht jedoch zu einem unerwünschten Überlaufen aus der Salzeinfüllöffnung heraus.

In einer weiteren Variante ist die Außenhülle des Salzbehälters teilweise oder vollständig in Form eines flexiblen Beutels ausgestaltet. Dabei können sowohl die Form als auch das von dem Beutel eingenommene Volumen in Abhängigkeit des Volumens der Salzsole und des Salzes, die sich beide in dem Beutel befinden, verändert bzw. angepasst werden. So ist es denkbar, dass der Beutel eine kompakte Form mit einem geringen Volumen aufweist, wenn sich nur wenig Salzsole in ihm befindet. Wird hingegen Salz nachgefüllt und damit das Gesamtvolumen aus Salz und Salzsole vergrößert, würde sich auch der Beutel dehnen und seine Form verändern. Dabei ist es denkbar und vorgesehen, das maximal mögliche Beutelvolumen durch eine starre Beutelaufnahme, in der der Beutel liegt, vorzugeben. Ferner ist es denkbar, dass die Außenhülle des Beutels zwar flexibel, aber nicht dehnbar ist. Dann ist das maximal mögliche Einfüllvolumen durch das Maximalvolumen des Beutels vorgegeben, wobei das vom Beutel eingenommene Volumen dann unterhalb des Maximalvolumens liegt, wenn auch weniger Salz und Salzsole in dem Beutel aufgenommen sind.

In einer weiteren Variante ist der Salzbehälter beweglich in dem Reinigungs- und Desinfektionsgerät angeordnet. Dabei kann der Salzbehälter von einer Betriebsposition in eine Befüllposition und von der Befüllposition wieder in die Betriebsposition überführt werden. In der Betriebsposition liegt der Salzbehälter dann vor, wenn kein Salz in ihn eingefüllt werden soll. Die Befüllposition dient hingegen einem möglichst einfachen Einfüllen von Salz in den Salzbehälter. In der Befüllposition ist die Salzeinfüllöffnung für einen Benutzer leicht zugänglich. Beispielsweise kann der Salzbehälter in dieser Variante an einem Ausziehmechanismus wie etwa in einer Schublade angeordnet sein, der aus dem Reinigungs- und Desinfektionsgerät heraus zu einem Benutzer hingezogen werden kann. Der Salzbehälter kann dann zwar nicht selbst aus dem Reinigungs- und Desinfektionsgerät herausgenommen werden, ist jedoch dennoch zum Einfüllen von Salz für einen Benutzer leicht zugänglich. Auch in dieser Variante kann die Außenhülle des Salzbehälters zumindest abschnittsweise flexibel ausgestaltet sein. Ferner ist es auch in dieser Variante denkbar, dass der Salzbehälter einen Beutel mit einer flexiblen Außenhülle aufweist oder vollständig aus einem solchen Beutel besteht. Allerdings ist es in dieser Variante insbesondere vorgesehen, dass der Salzbehälter vollständig eine feste, unflexible Außenhülle aufweist.

Die während des Einfüllens zu verdrängende Salzsole kann in dieser Variante direkt in den Abflussstrang des Reinigungs- und Desinfektionsgerät gepumpt werden. Dies kann beispielsweise mit einer Schlauchpumpe erfolgen. Dann wird ein Kontakt zwischen der aggressiven Salzsole und empfindlichen Gerätebestandteilen vermieden. Alternativ ist es auch denkbar, dass der Ausziehmechanismus, an dem der Salzbehälter angeordnet ist, aus einem korrosionsbeständigen Material gefertigt ist und zum Auffangen überlaufender Salzsole dient. Dann muss die Salzsole anschließend aus dem Ausziehmechanismus entfernt werden, was abermals über eine Pumpe erfolgen kann. Um unnötige Verschmutzungen des Ausziehmechanismus bzw. des Reinigungs- und Desinfektionsgeräts zu vermeiden, ist es in dieser Variante angeraten, eine Reinigungsvorrichtung für den entsprechenden Ausziehmechanismus vorzusehen.

Das Herausziehen des Salzbehälters aus dem Reinigungs- und Desinfektionsgerät, ohne den Salzbehälter dabei vollständig aus dem Reinigungs- und Desinfektionsgerät entfernen zu können, ist mit dem Vorteil verbunden, dass Schlauchanschlüsse zwischen den Salzbehälter und weiteren Elementen des Reinigungs- und Desinfektionsgeräts nicht gelöst werden brauchen, wenn Salz in den Salzbehälter eingefüllt wird. Vielmehr können diese Schlauchverbindungen derart bemessen sein, dass der Salzbehälter problemlos von der Betriebsposition in die Befüllposition und wieder zurück bewegt werden kann.

In einer weiteren Variante ist aber gerade das vollständige Entfernen des Salzbehälters aus dem Reinigungs- und Desinfektionsgerät vorgesehen. Dabei wird in dieser Variante nur der Salzbehälter aus dem Gerät entfernt, nicht jedoch andere, für den Betrieb des Geräts erforderlichen Bestandteile. Das vollständige Entfernen des Salzbehälters hat den Vorteil, dass dieser dann unabhängig vom Reinigungs- und Desinfektionsgerät an einem beliebigen, vom Benutzer gewählten Ort mit Salz befüllt werden kann. Beispielsweise kann dies über einem Waschbecken erfolgen, sodass beim Einfüllen des Salzes austretende Salzsole in das Waschbecken laufen und dort weggespült werden kann. Auch durch diese Variante wird ein Kontakt von empfindlichen Bestandteilen des Reinigungs- und Desinfektionsgerät mit der aggressiven Salzsole vermieden. Allerdings ist es in dieser Variante erforderlich, eine mit Ventilen versehene Andockvorrichtung zwischen dem Salzbehälter und weiteren Komponenten des Reinigungs- und Desinfektionsgeräts vorzusehen, um ein Überführen von Salzsole aus dem Salzbehälter zur Enthärtungseinrichtung zu ermöglichen. Dies kann beispielsweise über an der Rückseite des Salzbehälters ausgebildete Ventileinrichtungen, die in der Betriebsposition des Salzbehälters von geeignet ausgebildeten Verbindungseinrichtungen, die im Reinigungs- und Desinfektionsgerät angeordnet sind, in Eingriff genommen werden, um eine Strömungsverbindung zwischen einem Innenraum des Salzbehälters und der Enthärtungseinrichtung herstellen zu können.

In einer weiteren Variante ist der Salzbehälter fest im Reinigungs- und Desinfektionsgerät verbaut. Dabei ist er insbesondere unbeweglich im Reinigungs- und Desinfektionsgerät angeordnet. Eine derartige Ausgestaltung reduziert den Aufwand bei der Herstellung des Reinigungs- und Desinfektionsgeräts, da keine beweglichen Teile in Bezug auf den Salzbehälter vorgesehen und eingebaut werden müssen. Dennoch ist es in dieser Variante möglich, die Salzeinfüllöffnung derart zu positionieren, dass ein Benutzer sie einfach von der Gerätefront her bedienen kann.

Um das Einfüllen von Salz bei einem fest eingebauten Salzbehälter zu erleichtern, ist in einer Variante ein Salzeinfüllrohr zum Aufsetzen auf die Salzeinfüllöffnung vorgesehen. Dabei ist das Salzeinfüllrohr derart ausgebildet, dass es in seinem auf die Salzeinfüllöffnung aufgesetzten Zustand zumindest abschnittsweise schräg nach oben (beispielsweise zum Benutzer hin) weist. Wenn ein Benutzer dann an dem ihm zugewandten Ende des Salzeinfüllrohrs Salz in das Salzeinfüllrohr füllt, fällt dieses aufgrund der Schwerkraft automatisch durch das Salzeinfüllrohr zur Salzeinfüllöffnung und in den Salzbehälter. Um einem Benutzer das Einfüllen von Salz weiter zu erleichtern, kann ein Salzeinfülltrichter an der ihm zugewandten Öffnung des Salzeinfüllrohrs vorgesehen sein. Dieser Salzeinfülltrichter kann ein integraler Bestandteil des Salzeinfüllrohrs sein oder aber als separates Bauteil auf das Salzeinfüllrohr aufsteckbar sein.

Wie bereits oben erwähnt, ist insbesondere bei Salzbehältern mit einer starren, unflexiblen Außenhülle dafür Sorge zu tragen, dass die beim Einfüllen des Salzes in den Salzbehälter verdrängte Salzsole nicht mit empfindlichen Teilen des Reinigungs- und Desinfektionsgeräts in Kontakt kommt und auch sonst keine bleibenden Verschmutzungen hinterlässt. In einer Variante ist daher eine besondere Ausgestaltung des Salzbehälters vorgesehen. So weist der Salzbehälter in dieser Variante eine Hauptkammer auf, in der sich das Salz und die Salzsole befinden. Ferner weist er eine Nebenkammer auf, die strömungstechnisch mit der Hauptkammer verbunden ist, im gewöhnlichen Betriebszustand des Salzbehälters jedoch nicht mit Salzsole befüllt ist. Wenn nun Salz durch die Salzeinfüllöffnung in den Salzbehälter gefüllt wird, dient die Nebenkammer dazu, Salzsole aus der Hauptkammer aufzufangen. Die Nebenkammer dient folglich als Überlauf für verdrängte Salzsole. Die in der Nebenkammer aufgefangene Salzsole kann anschließend durch eine Pumpe, beispielsweise eine Schlauchpumpe, abgepumpt werden, sodass das volle Volumen der Nebenkammer bei einem späteren Salzeinfüllvorgang erneut zur Verfügung steht. Sich dabei innerhalb des Salzbehälters möglicherweise ansammelnde Salzablagerungen sind für die Gerätesauberkeit und -sicherheit insgesamt unkritisch, da sich im Salzbehälter im Laufe seiner gesamten Betriebsdauer ohnehin Salzablagerungen ergeben können.

Das Vorsehen einer Hauptkammer und einer Nebenkammer in dem Salzbehälter ist nicht nur dann möglich und vorgesehen, wenn dieser eine vollständig starre, unflexible Hülle aufweist. Vielmehr ist eine solche Ausgestaltung auch in Varianten denkbar, die eine zumindest abschnittsweise flexible Außenhülle des Salzbehälters vorsehen.

Die Salzeinfüllöffnung kann grundsätzlich jede beliebige Form aufweisen. In einer Variante ist die Salzeinfüllöffnung kreisrund ausgebildet. Dann ist es besonders einfach möglich, handelsübliche Trichter auf die Salzeinfüllöffnung aufzusetzen, um Salz noch einfacher in den Salzbehälter einzufüllen.

In allen der vorstehend erläuterten Varianten wird ein direkter Kontakt mit während des Einfüllens von Salz aus dem Salzbehälter austretender Salzsole mit empfindlichen Teilen des Reinigungs- und Desinfektionsgeräts vermieden. Dadurch wird die Lebensdauer des Geräts und seiner Komponenten signifikant erhöht, was die Nachhaltigkeit des beanspruchten Reinigungs- und Desinfektionsgeräts ebenfalls erhöht.

Die der Erfindung zugrunde liegende Aufgabe wird auch durch ein Verfahren mit den Merkmalen des Anspruchs 14 gelöst. Ein solches Verfahren dient zum Betrieb eines Reinigungs- und Desinfektionsgeräts für medizinische und/oder zahnmedizinische Instrumente. Dabei weist das Reinigungs- und Desinfektionsgerät eine an einer Gerätefront angeordnete Gerätetür auf, durch die hindurch eine Waschkammer des Geräts zugänglich ist. Ferner weist das Gerät eine Wasserenthärtungseinrichtung und einen Salzbehälter auf. In dem Salzbehälter ist eine Salzsole enthalten, die zu einer Regeneration eines in der Wasserenthärtungseinrichtung befindlichen Enthärtungsmittels dient. In einer Variante kann die Salzsole im Salzbehälter hergestellt werden.

Das erfindungsgemäß beanspruchte Verfahren zeichnet sich dadurch aus, dass der Salzbehälter eine von einer Außenseite der Gerätefront des Reinigungs- und Desinfektionsgeräts zugängliche Salzeinfüllöffnung aufweist. Durch diese Salzeinfüllöffnung hindurch kann Salz in den Salzbehälter eingefüllt werden. Die Gerätetür muss hierfür nicht geöffnet werden. Das Verfahren sorgt dabei dafür, dass ein Einfüllen von Salz in die Salzeinfüllöffnung unabhängig vom konkreten Betriebszustand des Reinigungs- und Desinfektionsgeräts möglich ist. So kann Salz während eines Programmlaufs des Reinigungs- und Desinfektionsgeräts in die Salzeinfüllöffnung eingefüllt werden. Alternativ kann das Salz auch in einem ausgeschalteten Zustand des Reinigungs- und Desinfektionsgeräts eingefüllt werden. Dabei sorgt das Verfahren dafür, dass eine zeitweise Entnahme des Salzbehälters aus dem Gerät oder das Überführen des Salzbehälters von einem Betriebszustand in einen Befüllzustand in Bezug auf die Durchführung des von dem Reinigungs- und Desinfektionsgerät durchgeführten Programms unkritisch ist.

Sämtliche der vorstehend erläuterten Varianten und Ausführungsbeispiele können in beliebiger Weise miteinander kombiniert werden. Darüber hinaus sind alle Varianten und Ausgestaltungen einzeln oder in beliebiger Kombination von dem beschriebenen Reinigungs- und Desinfektionsgerät auf das beschriebene Verfahren übertragbar, und umgekehrt.

Die vorliegende Erfindung wird anhand von Figuren und Ausführungsbeispielen nachfolgend näher erläutert werden. Es zeigen:
- Figur 1: ein erstes Ausführungsbeispiel eines Reinigungs- und Desinfektionsgeräts mit einem von außen zugänglichen Salzbehälter und
- Figur 2: ein zweites Ausführungsbeispiel eines Reinigungs- und Desinfektionsgerät mit einem von außen zugänglichen Salzbehälter.

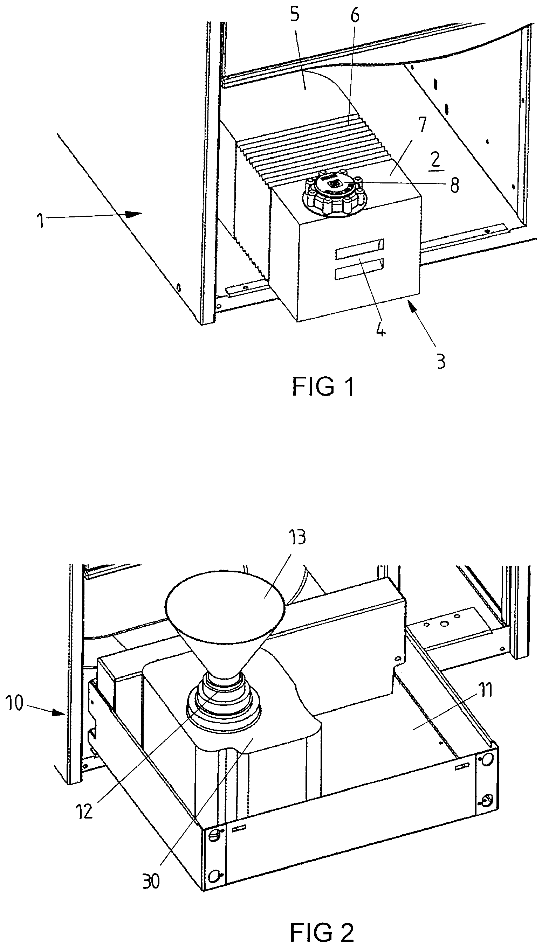

Die Figur 1 zeigt den unteren Bereich eines Reinigungs- und Desinfektionsgeräts 1, bei dem eine in der Gerätefront des Reinigungs- und Desinfektionsgeräts 1 vorhandene Klappe entfernt ist. Dadurch ist es möglich, von der Gerätefront aus in das Innere des Reinigungs- und Desinfektionsgerät 1 zu sehen. Dort ist auf dem Boden 2 des Reinigungs- und Desinfektionsgeräts 1 ein Salzbehälter 3 angeordnet, der zur Aufnahme von Regeneriersalz dient. Aus diesem Regeneriersalz wird zusammen mit Wasser eine Salzsole hergestellt, die dann zur Regenerierung eines in einer Wasserenthärtungseinrichtung des Reinigungs- und Desinfektionsgeräts 1 enthaltenen Enthärtungsmittels eingesetzt wird. An seiner der Gerätefront zugewandten Vorderseite weist der Salzbehälter 3 einen Handgriff 4 auf, mit dem er von einem vor der Gerätefront stehenden Benutzer zu sich hin gezogen werden kann.

Der Salzbehälter 3 weist einen ersten Teil 5 auf, der einen hinteren Bereich des Salzbehälters 3 darstellt und der fest mit dem Reinigungs- und Desinfektionsgerät 1 verbunden ist. Dieser erste Teil 5 weist eine starre, unflexible Außenhülle, beispielsweise aus einem Kunststoff wie etwa Polyethylen oder Polypropylen, auf. Der erste Teil 5 des Salzbehälters 3 geht nahtlos in einen dritten Teil 6 des Salzbehälters 3 über. Dieser dritte Teil 6, der auch als Mittelteil bezeichnet werden kann, ist als Faltenbalg ausgestaltet und stellt ein längenveränderliches Element des Salzbehälters 3 dar. Der dritte Teil 6 geht ebenfalls nahtlos in einen zweiten Teil 7 des Salzbehälters 3 über, der abermals - wie der erste Teil 5 des Salzbehälters 3 - eine starre, unflexible Außenhülle aufweist. Der zweite Teil 7 des Salzbehälters 3 bildet den vorderen Bereich des Salzbehälters 3. In ihm ist neben dem bereits erwähnten Griff 4 ein Deckel 8 ausgebildet, der eine darunterliegende Salzeinfüllöffnung verschließt. Die Salzeinfüllöffnung dient dafür, Salz in den Salzbehälter 3 einzufüllen.

Üblicherweise befindet sich der Salzbehälter 3 vollständig im Innenraum des Reinigungs- und Desinfektionsgeräts 1. Wenn - wie in der Figur 1 dargestellt - eine im unteren Bereich der Gerätefront angeordnete Klappe entfernt ist, kann der Salzbehälter 3 durch Ausübung einer Zugkraft auf den Griff 4 zumindest teilweise aus dem Reinigungs- und Desinfektionsgerät 1 herausgezogen werden. Ein vollständiges Herausziehen des Salzbehälters 3 ist nicht möglich, da - wie erwähnt - der erste Teil 5 des Salzbehälters 3 fest mit dem Reinigungs- und Desinfektionsgerät 1 verbunden ist. Aber insbesondere der zweite Teil 7 des Salzbehälters 3 lässt sich so weit aus dem Reinigungs- und Desinfektionsgerät herausziehen, dass die Salzeinfüllöffnung von einer Außenseite des Reinigungs- und Desinfektionsgeräts 1 aus zugänglich ist, wie dies in der Figur 1 dargestellt ist.

Das teilweise Herausziehen des Salzbehälters 3 hat zur Folge, dass sich der Faltenbalg des dritten Teils 6 des Salzbehälters 3 dehnt und dass sich das Innenvolumen des Salzbehälters 3 vergrößert. Wenn nun Salz durch die Salzeinfüllöffnung in den Salzbehälter 3 eingefüllt wird, kommt es nicht zum Überlaufen von durch das eingefüllte Salz verdrängter Salzsole. Vielmehr steigt der Spiegel der Salzsole durch das neu eingefüllte Salz lediglich wieder bis unterhalb der Salzeinfüllöffnung an. Dann kann der Deckel 8 auf die Salzeinfüllöffnung geschraubt werden. Wenn der Salzbehälter 3 anschließend wieder durch Ausübung einer Druckkraft auf den Griff 4 vollständig in das Reinigungs- und Desinfektionsgerät 1 eingeschoben wird, wird ein Teil der sich im Inneren des Salzbehälters 3 befindlichen Salzsole durch das verringerte zur Verfügung stehende Volumen durch eine entsprechende Schlauchverbindung aus dem Salzbehälter 3 herausgedrückt und beispielsweise direkt einem Abflussstrang zugeführt.

Wenn der Salzbehälter 3 wieder vollständig in dem Reinigungs- und Desinfektionsgerät 1 aufgenommen ist, kann die Abdeckplatte zum Abdecken des unteren Bereichs der Gerätefront des Reinigungs- und Desinfektionsgeräts 1 wieder eingesetzt werden, sodass der Salzbehälter 3 nicht mehr von außen sichtbar ist.

Wenn sich der Salzbehälter 3 vollständig in dem Reinigungs- und Desinfektionsgerät 1 befindet, kann dies auch als Betriebsposition des Salzbehälters 3 bezeichnet werden. Wenn der Salzbehälter 3 teilweise aus dem Reinigungs- und Desinfektionsgerät 1 herausgezogen ist, um eine leichte Zugänglichkeit der unter dem Deckel 8 befindlichen Salzeinfüllöffnung zu gewährleisten, kann dies auch als Befüllposition des Salzbehälters 3 bezeichnet werden. Die Figur 2 zeigt ein weiteres Ausführungsbeispiel eines Salzbehälters 30, der in einem Reinigungs- und Desinfektionsgerät 10 angeordnet ist. Dabei ist in der Figur 2 wiederum nur ein unterer Bereich des Reinigungs- und Desinfektionsgeräts 10 zu sehen. Der Salzbehälter 30 ist in einer Schublade 11 aufgenommen, die als Ausziehmechanismus dient und die in einem bodennahen Bereich des Reinigungs- und Desinfektionsgerät 10 angeordnet ist und gegenüber dem Gehäuse des Reinigungs- und Desinfektionsgerät 10 beweglich ausgestaltet ist. So kann die Schublade 11 aus dem Gehäuse des Reinigungs- und Desinfektionsgeräts 10 herausgezogen werden, um eine leichte Zugänglichkeit einer Einfüllöffnung 12 des Salzbehälters 30 zu ermöglichen. Um das Einfüllen von Salz durch die Einfüllöffnung 12 in den Salzbehälter 30 weiter zu vereinfachen, ist ein Trichter 13 vorgesehen, der auf die Einfüllöffnung 12 aufgesetzt ist. In der Betriebsposition des Salzbehälters 30, wenn die Schublade vollständig innerhalb des Reinigungs- und Desinfektionsgeräts 10 angeordnet ist, ist die in der Figur 2 dargestellte Öffnung in der Gerätefront des Reinigungs- und Desinfektionsgeräts 10 durch eine Klappe verdeckt. Wenn diese Klappe entfernt wird, kann die Schublade 11 aus dem Gehäuse des Reinigungs- und Desinfektionsgeräts 10 herausgezogen werden, wie dies in der Figur 2 dargestellt ist. Dann befindet sich der Salzbehälter 30 in seiner Befüllposition. Auch in dieser Befüllposition ist ein weiterer Betrieb des Reinigungs- und Desinfektionsgeräts 10 problemlos möglich, da der Salzbehälter 30 auch in der Befüllposition über rückwärtige angeschlossene Schlauchleitungen mit weiteren Komponenten des Reinigungs- und Desinfektionsgeräts 10 verbunden ist und insbesondere eine strömungstechnische Verbindung mit einer Enthärtungseinrichtung des Reinigungs- und Desinfektionsgeräts 10 realisiert ist.

Der Salzbehälter 30 ist dabei fest in der Schublade 11 montiert, die wiederum nicht dafür vorgesehen ist, aus dem Reinigungs- und Desinfektionsgerät 10 entnommen zu werden. Damit lässt sich der Salzbehälter 30 mit Hilfe der Schublade 11 lediglich von seiner Betriebsposition in seine Befüllposition und wieder zurück in seine Betriebsposition verfahren, um ein einfaches Befüllen des Salzbehälters 30 mit Salz zu ermöglichen.

Bei dem in der Figur 2 dargestellten Ausführungsbeispiel wird ein Austreten von in dem Salzbehälter 30 befindlicher Salzsole beim Nachfüllen von Salz durch ein Abpumpen der Salzsole über eine an der Rückseite des Salzbehälters 30 angeschlossene Schlauchpumpe sichergestellt.

## Patentansprüche

1. Reinigungs- und Desinfektionsgerät für medizinische und/oder zahnmedizinische Instrumente, mit einer an einer Gerätefront angeordneten Gerätetür, einer Wasserenthärtungseinrichtung und einem Salzbehälter (3, 30), in welchem eine Salzsole enthalten sein kann, die zu einer Regeneration eines in der Wasserenthärtungseinrichtung befindlichen Enthärtungsmittels dient,
**dadurch gekennzeichnet,**
**dass** der Salzbehälter (3, 30) eine von einer Außenseite der Gerätefront des Reinigungs- und Desinfektionsgeräts (1, 10) zugängliche Salzeinfüllöffnung (12) aufweist, durch die hindurch Salz in den Salzbehälter (3, 30) eingefüllt werden kann, ohne dass die Gerätetür hierfür geöffnet werden muss, wobei ein Einfüllen von Salz in die Salzeinfüllöffnung (12) während eines Programmlaufs des Reinigungs- und Desinfektionsgeräts (1, 10) oder in einem ausgeschalteten Zustand des Reinigungs- und Desinfektionsgeräts (1, 10) möglich ist.

2. Reinigungs- und Desinfektionsgerät nach Anspruch 1, **dadurch gekennzeichnet, dass** die Salzeinfüllöffnung (3, 30) hinter einer Klappe in der Gerätefront angeordnet ist.

3. Reinigungs- und Desinfektionsgerät nach Anspruch 2, **dadurch gekennzeichnet, dass** die Klappe werkzeugfrei geöffnet werden kann.

4. Reinigungs- und Desinfektionsgerät nach einem der vorhergehenden Ansprüche, **dadurch gekennzeichnet, dass** der Salzbehälter (3, 30) eine zumindest abschnittsweise flexible Außenhülle aufweist.

5. Reinigungs- und Desinfektionsgerät nach einem der vorhergehenden Ansprüche, **dadurch gekennzeichnet, dass** der Salzbehälter einen fest in dem Reinigungs- und Desinfektionsgerät angeordneten ersten Teil (5) und einen gegenüber dem ersten Teil beweglichen zweiten Teil (7) aufweist, in dem die Salzeinfüllöffnung angeordnet ist.

6. Reinigungs- und Desinfektionsgerät nach Anspruch 5, **dadurch gekennzeichnet, dass** zwischen dem ersten Teil (5) und dem zweiten Teil (7) ein längenvariabler dritter Teil (6) des Salzbehälters (3) angeordnet ist.

7. Reinigungs- und Desinfektionsgerät nach Anspruch 6, **dadurch gekennzeichnet, dass** der dritte Teil (6) des Salzbehälters (3) als Faltenbalg ausgestaltet ist.

8. Reinigungs- und Desinfektionsgerät nach einem der Ansprüche 1 bis 4, **dadurch gekennzeichnet, dass** der Salzbehälter (3, 30) einen Beutel mit einer flexiblen Außenhülle aufweist, wobei eine Form des Beutels und ein von dem Beutel eingenommenes Volumen in Abhängigkeit eines Volumens von in dem Salzbehälter (3, 30) befindlichen Salzes und befindlicher Salzsole veränderlich sind.

9. Reinigungs- und Desinfektionsgerät nach einem der Ansprüche 1 bis 4, **dadurch gekennzeichnet, dass** der Salzbehälter (3, 30) beweglich in dem Reinigungs- und Desinfektionsgerät angeordnet ist und von einer Betriebsposition in eine Befüllposition überführt werden kann, in der die Salzeinfüllöffnung (12) für einen Benutzer leicht zugänglich ist.

10. Reinigungs- und Desinfektionsgerät nach Anspruch 9, **dadurch gekennzeichnet, dass** der Salzbehälter (3, 30) vollständig aus dem Reinigungs- und Desinfektionsgerät entfernt werden kann.

11. Reinigungs- und Desinfektionsgerät nach einem der Ansprüche 1 bis 4, **dadurch gekennzeichnet, dass** der Salzbehälter (3, 30) fest im Reinigungs- und Desinfektionsgerät verbaut ist.

12. Reinigungs- und Desinfektionsgerät nach Anspruch 11, **dadurch gekennzeichnet, dass** ein Salzeinfüllrohr zum Aufsetzen auf die Salzeinfüllöffnung (12) vorgesehen ist, das in seinem auf die Salzeinfüllöffnung (12) aufgesetzten Zustand zumindest abschnittsweise eine von der Salzeinfüllöffnung (12) schräg nach oben weisende Ausrichtung aufweist.

13. Reinigungs- und Desinfektionsgerät nach einem der vorherigen Ansprüche, **dadurch gekennzeichnet, dass** der Salzbehälter (3, 30) eine Hauptkammer und eine mit der Hauptkammer strömungstechnisch verbundene Nebenkammer aufweist, die dem Auffangen von Salzsole aus der Hauptkammer dient, wenn Salz in die Hauptkammer des Salzbehälters (3, 30) eingefüllt wird.

14. Verfahren zum Betrieb eines Reinigungs- und Desinfektionsgeräts für medizinische und/oder zahnmedizinische Instrumente, wobei das Reinigungs- und Desinfektionsgerät (1, 10) eine an einer Gerätefront angeordnete Gerätetür, eine Wasserenthärtungseinrichtung und einen Salzbehälter (3, 30) aufweist, in welchem eine Salzsole enthalten ist, die zu einer Regeneration eines in der Wasserenthärtungseirichtung befindlichen Enthärtungsmittels dient,
**dadurch gekennzeichnet,**
**dass** der Salzbehälter (3, 30) eine von einer Außenseite der Gerätefront des Reinigungs- und Desinfektionsgeräts (1, 10) zugängliche Salzeinfüllöffnung (12) aufweist, durch die hindurch Salz in den Salzbehälter (3, 30) eingefüllt werden kann, ohne dass die Gerätetür hierfür geöffnet werden muss, wobei das Verfahren ein Einfüllen von Salz in die Salzeinfüllöffnung (12) während eines Programmlaufs des Reinigungs- und Desinfektionsgeräts (1, 10) oder in einem ausgeschalteten Zustand des Reinigungs- und Desinfektionsgeräts gestattet.
